# DEMANDE DE BREVET EUROPEEN

(11) **EP 3 461 917 A1**
(43) Date de publication de la demande: **03.04.2019**
(21) Numéro de dépôt: 18191591.9
(22) Date de dépôt: 30.08.2018
(51) Int. Cl.: C12R 1/685, C12R 1/69, C12N 1/14, C12R 1/66

(54) **PROCEDE DE MODIFICATION DE LA MORPHOLOGIE DE CHAMPIGNONS FILAMENTEUX AGREGES DE TYPE COAGULANT**

(30) Priorité: 27.09.2017 FR 1758924
(71) Demandeur: IFP Energies nouvelles, 92852 Rueil-Malmaison Cedex (FR)
(72) Inventeur: DROUGARD, Marion, 92500 Rueil Malmaison (FR); ANDRE, Estelle, 92500 Rueil Malmaison (FR); JOURDIER, Etienne, 92420 Vaucresson (FR); BEN CHAABANE, Fadhel, 75020 Paris (FR); PANNACCI, Nicolas, 75018 Paris (FR)
(74) Mandataire: IFP Energies nouvelles

(57) **Abrégé**

L'invention a pour objet un procédé de modification de la morphologie de champignons filamenteux agrégés de type coagulant comprenant:
a) l'encapsulation des spores desdits champignons filamenteux dans la phase dispersée d'une émulsion eau-dans-huile,
b) la germination des spores encapsulées dans ladite phase dispersée de l'émulsion,
c) la récupération des spores germées non agrégées,
ladite émulsion étant obtenue par mise en oeuvre d'un dispositif microfluidique,
d) la mise en culture des spores germées en milieu liquide.

## Description

La présente invention a pour objet un procédé de modification de la morphologie de champignons filamenteux agrégés de type coagulant par encapsulation des spores dans une émulsion microfluidique.

Les champignons filamenteux sont couramment utilisés pour produire des enzymes, susceptibles, par exemple, de catalyser la transformation de biomasse en éthanol dans le cadre de la fabrication de bio-carburants, ou de fabriquer différents acides utiles dans l'industrie agroalimentaire. *Aspergillus oryzae* permet par exemple de produire plusieurs enzymes (cellulases, amylases) utiles à l'industrie de production de biocarburants mais également pour la chimie et l'alimentaire (exemple : l'asparaginase qui permet de réduire le taux d'acrylamide dans les aliments). Plusieurs publications récentes ont aussi montré son intérêt potentiel pour la production d'acides dicarboxyliques en C4 (malate, fumarate, succinate).

La morphologie des champignons filamenteux est susceptible d'impacter directement la productivité des souches, principalement en modifiant le transport de l'oxygène et des nutriments au sein du mycélium. On distingue classiquement deux types de morphologie pour ces champignons : une morphologie non agrégée nommée mycélium libre, et une morphologie agrégée pouvant se présenter sous la forme de plusieurs agrégats sphériques, nommés pellets ou sous la forme d'un seul agrégat. Selon les espèces de champignons considérées, l'agrégation peut se produire à plusieurs stades de la croissance, à des stades dit « précoces » avant et pendant la germination des spores ou à des stades plus avancés lorsque le mycélium est déjà formé. Ainsi, les espèces dont l'agrégation est effective entre les spores en germination sont appelées espèce de type « coagulants » alors que celles s'agrégeant plus tardivement uniquement lors de la croissance du mycélium sont regroupées sous les espèces de type « non-coagulant ».

Les champignons se présentant sous des formes agrégées présentent des limitations diffusionnelles qui affectent leur productivité. Le contrôle de la morphologie des champignons filamenteux pour garantir l'obtention de mycéliums libres, constituerait donc un avantage significatif pour la maîtrise des procédés de production d'enzymes mettant en oeuvre ces champignons, afin d'améliorer leur productivité et de faciliter leur culture en bioréacteur. Il n'existe à ce jour dans la littérature aucune étude relative au contrôle de la morphologie des champignons filamenteux présentant une morphologie agrégée par mise en oeuvre de techniques microfluidiques.

### Résumé de l'invention

La présente invention propose l'utilisation de la microfluidique afin d'isoler physiquement, au sein d'une émulsion, les spores de champignons filamenteux au cours de leur germination. A la fin de la germination, la déstabilisation de l'émulsion, la récupération des tubes germinatifs puis leur mise en culture permet d'obtenir une croissance fluide pour certaines espèces ayant habituellement une croissance sous forme agrégée.

La croissance sous forme fluide facilite la mise en culture des champignons et permet d'augmenter les productivités des procédés les mettant en oeuvre.

L'invention a ainsi pour objet un procédé de modification de la morphologie de champignons filamenteux agrégés de type coagulant comprenant:
a) l'encapsulation des spores desdits champignons filamenteux dans la phase dispersée d'une émulsion eau-dans-huile,
b) la germination des spores encapsulées dans ladite phase dispersée de l'émulsion,
c) la récupération des spores germées non agrégées,
   ladite émulsion étant obtenue par mise en oeuvre d'un dispositif microfluidique,
d) la mise en culture des spores germées en milieu liquide.

Au sens de la présente demande, la microfluidique est définie comme la technologie des systèmes qui manipulent de petits volumes de fluides (10⁻⁹ à 10⁻¹⁸ litres), en utilisant des canaux de la dimension de quelques dizaines de micromètres.

Beneyton et al. a décrit en 2016 l'utilisation de la microfluidique pour l'encapsulation des spores de l'espèce Aspergillus niger pour le criblage enzymatique haut-débit de souches mutantes par fluorescence. Un grand nombre de spores soumises à une mutagénèse aléatoire sont encapsulées séparément au sein de gouttes de milieu de culture, dispersée dans une phase continue d'huile. Au cours de leur croissance, la dégradation d'un substrat fluorescent contenu dans les gouttes permet d'évaluer la production d'enzymes par le champignon encapsulé et ainsi de sélectionner parmi la population les souches mutantes les plus performantes. Beneyton et al. ne décrit pas la mise en oeuvre de cette technologie pour modifier la morphologie de champignons filamenteux.

Le procédé selon l'invention permet la modification de la morphologie de champignons filamenteux qui, naturellement forment des agrégats par regroupement des spores au cours de leur développement (= type coagulant). La présente invention a pour objet de prévenir, voire de supprimer cette agrégation en isolant physiquement les spores les unes des autres avant et pendant la germination, par leur encapsulation dans une émulsion eau-dans-huile obtenue par microfluidique.

### Description des figures

La Figure 1 illustre une géométrie typique utilisée pour la formation de gouttes à une jonction en « flow-focusing ».
La Figure 2 illustre un dispositif microfluidique mis en oeuvre dans les exemples de l'invention.
La Figure 3 est une observation microscopique de l'encapsulation des spores réalisée en mettant en oeuvre le procédé de l'invention.
La Figure 4 est une série de photographies d'Erlenmeyer de culture d'*Aspergillus oryzae* observées dans les conditions de l'exemple 2 : 4A) et 4B) illustrent une culture témoin, 4C) illustre une culture de spores issues du procédé d'encapsulation de l'invention.
La Figure 5 représente des photographies en loupe binoculaire de culture *d'Aspergillus oryzae* observées dans les conditions de l'exemple 2 : 5A) échantillon de la culture témoin, 5C) échantillon de la culture issue du procédé d'encapsulation de l'invention.
La Figure 6 est une série de photographies d'Erlenmeyer de culture *d'Aspergillus terreus* observées dans les conditions de l'exemple 3 : 6A) et 6B) illustrent une culture témoin, 6C) illustre une culture de spores issues du procédé d'encapsulation de l'invention.
La Figure 7 représente des photographies en loupe binoculaire de culture *d'Aspergillus terreus* observées dans les conditions de l'exemple 3 : 7A) échantillon de la culture témoin, 7C) échantillon de la culture issue du procédé d'encapsulation de l'invention.

### Champignon filamenteux de morphologie agrégée

Dans le cadre de la présente demande, on entend par champignon filamenteux de morphologie agrégée appartenant au type coagulant, un champignon dont les spores non germées et en cours de germination s'agrègent naturellement entre elles.

Selon un mode préféré de réalisation, le champignon filamenteux de morphologie agrégée est choisi parmi les espèces *Aspergillus oryzae, Aspergillus niger ou Aspergillus nidulans*, et plus préférentiellement parmi les souches appartenant à l'espèce *Aspergillus oryzae.*

Dans le cadre du présent procédé, on utilise des spores de champignons filamenteux. Les spores peuvent être filtrées avant utilisation dans le procédé de l'invention, afin de s'affranchir de la présence éventuelle de mycélium.

### Encapsulation

Le procédé de modification de la morphologie des champignons filamenteux selon l'invention comprend une première étape d'encapsulation des spores du champignon dans la phase dispersée d'une émulsion eau-dans-huile.

L'émulsion est obtenue par mise en oeuvre d'un dispositif microfluidique.

La phase dispersée de l'émulsion est une phase aqueuse comprenant au moins de l'eau, des spores de champignon à encapsuler en suspension dans un milieu de culture liquide.

Le milieu de culture peut être tout milieu connu de l'homme du métier pour permettre la germination des champignons filamenteux objets de la présente demande. Il peut s'agir par exemple d'un milieu riche contenant des sources de nutriments complexes (par exemple : extrait de levure, peptone) ou d'un milieu minimum contenant des composés définis (par exemple : sels minéraux, composés carbonés et azotés).

Il peut s'agir par exemple d'un milieu Yeast Extract-Peptone-Dextrose comprenant 24g/L de dextrose de pomme de terre, 5g/L de peptone et 5g/L d'extrait de levure.

La phase huileuse de l'émulsion comprend au moins une huile biocompatible n'interférant pas dans la croissance de micro-organisme. En particulier, la phase huileuse comprend au moins une huile fluorée. Les huiles fluorées sont en effet connues pour être biocompatibles pour des utilisations avec des microorganismes (voir par exemple Baret J.-C., Surfactants in droplet-based microfluidics, Lab on a chip, 2012, 12(3), p. 422).

L'huile fluorée peut être choisie parmi les perfluoroalcanes, les perfluoroamines et les perfluoroéthers. De préférence, ladite huile fluorée peut être choisie parmi le perfluoropentane, le perfluorohexane, le perfluorotripropylamine et le perfluoropolyéther.

Selon un mode préféré de réalisation, la phase huileuse comprend une huile fluorée, par exemple celle commercialisée par la société 3M sous la dénomination Fluorinert FC-3283.

Outre l'huile, la phase huileuse comprend au moins un tensioactif permettant la stabilisation des gouttes de phase aqueuse dispersées dans la phase huileuse. Le tensioactif peut, de préférence, être choisi parmi les tensioactifs biocompatibles. De préférence, le tensioactif est fluoré, tel que celui commercialisé par la société Dolomite sous la dénomination Picosurf.

L'émulsion selon l'invention est obtenue par microfluidique, c'est-à-dire par mise en oeuvre d'un système miniaturisé permettant de générer en flux des microgouttes de façon reproductible et contrôlée au sein de microcanaux agencés selon une géométrie particulière.

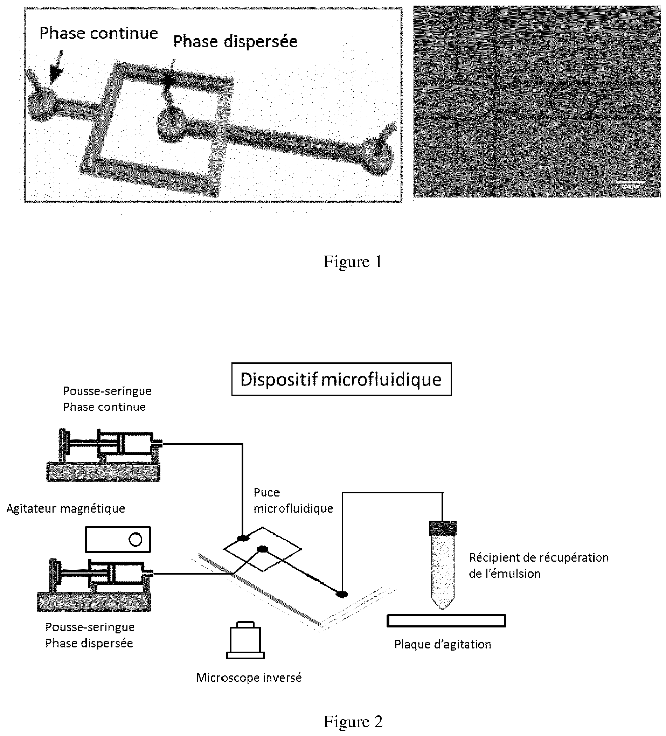

Ces géométries sont bien connues de l'homme de l'art. On peut citer, parmi les méthodes les plus classiques pour générer un flux de microgouttes, la jonction en T et la géométrie de focalisation de flux (« flow-focusing » en terminologie anglo-saxone). Selon un mode préféré de réalisation, l'émulsion selon l'invention est obtenue au moyen d'un système microfluidique présentant une géométrie « flow-focusing ». Dans ce type de système, la phase dispersée est injectée colinéairement à deux flux d'huile l'entourant. Selon les conditions expérimentales choisies, un jet de phase dispersée peut se former entre les deux flux d'huile. Le co-flux laminaire ainsi créé passe à travers une constriction, ce qui l'affine et induit éventuellement la rupture du jet de phase dispersée en gouttelettes. Dans la géométrie « flow-focusing », les gouttes sont donc de préférence formées au niveau d'une jonction en croix ou similaire. Ce type de système est par exemple illustré à la Figure 1.

Le dispositif microfluidique selon l'invention comprend notamment :
- un premier conduit microfluidique, alimenté en phase aqueuse contenant en suspension les spores à encapsuler ;
- un deuxième conduit microfluidique alimenté en phase huileuse;
   le premier conduit débouchant dans le deuxième conduit et formant une jonction fluidique avec ce dernier.

Les deux phases liquides n'étant pas miscibles, une interface se forme au niveau de la jonction fluidique, correspondant à l'intersection des conduits microfluidiques, permettant ainsi la formation de gouttes de phase aqueuse dispersées dans la phase huileuse, c'est-à-dire la formation de l'émulsion. Les pressions et débits des deux phases liquides sont dimensionnés de telle manière que l'interface reste stable par équilibre entre lesdites pressions et la tension interfaciale.

Le dispositif microfluidique selon l'invention comprend, de préférence, également au moins un conduit microfluidique d'évacuation de l'émulsion formée.

Selon un mode préféré de réalisation, le dispositif microfluidique présente une géométrie planaire, en particulier le dispositif selon l'invention est une puce microfluidique.

Les premier et deuxième conduits microfluidiques du système microfluidique mis en oeuvre, en particulier les conduits de la puce microfluidique utilisée, ont, de préférence, une section pouvant s'inscrire dans un cercle de diamètre compris entre 1 µm et 1 mm, de préférence entre 50 et 500 µm, et en particulier de l'ordre de 125 µm.

La phase aqueuse de l'émulsion obtenue au moyen du dispositif selon l'invention se présente sous la forme de gouttes dispersées dans la phase huileuse, lesdites gouttes présentant un diamètre compris entre 10 µm et 1 mm, de préférence entre 50 et 500 µm, et plus préférentiellement entre environ 140 et 150 µm. Le diamètre des gouttes peut, par exemple être mesuré par toute méthode connue de l'homme du métier, et notamment par analyse d'image sur un microscope optique.

Un tel dispositif ou une telle puce microfluidique peut être fabriqué à partir d'un substrat en silicium, silice ou verre par des techniques classiques de photolithographie et gravure empruntées à la microélectronique. En variante, le dispositif peut être réalisé en matière plastique (notamment en polydimethylsiloxane - PDMS) par moulage, à partir d'un moule, réalisé à son tour par photolithographie.

Le dispositif microfluidique selon l'invention peut avantageusement être traité hydrophobe, par exemple par traitement à l'OTS (octadécyltrichlorosilane).

Le dispositif microfluidique d'encapsulation peut, en outre, comprendre :
- un moyen d'injection, dans le premier conduit dudit dispositif, d'une suspension formée par la phase aqueuse et par les spores à encapsuler ; il peut s'agir par exemple d'une seringue avec un pousse-seringue ou d'un contrôleur en pression ; et
- un moyen d'injection, dans le premier conduit dudit dispositif, de la phase huileuse ; il peut s'agir également d'une seringue avec un pousse-seringue ou d'un contrôleur en pression.

Dans le cadre de la présente invention, la concentration de spores dans la phase aqueuse et les débits d'injection des phases sont adaptés de manière à avoir en moyenne entre 1 et 10 spores par goutte, de préférence 1 spore par goutte.

En particulier, la phase aqueuse comprenant les spores peut être introduite dans le dispositif microfluidique à un débit compris entre 0,1 et 20 mL/h, de préférence entre 1 et 10 mL/h, et plus préférentiellement de l'ordre de 3 mL/h.

La phase huileuse contenant le tensioactif peut être introduite dans le système microfluidique à un débit compris entre 1 et 50 mL/h, de préférence entre 5 et 20 mL/h, et plus préférentiellement de l'ordre de 8 mL/h.

L'étape d'encapsulation du procédé selon l'invention peut durer au moins 30 minutes, de préférence de 30 min à 10h, de préférence encore de 1 à 5h, et plus préférentiellement environ 3h.

L'émulsion formée est récupérée en sortie du dispositif microfluidique par le conduit microfluidique de sortie. Le conduit microfluidique de sortie présente une section pouvant s'inscrire dans un cercle de diamètre compris entre 125 et 1 500µm, de préférence entre 500 et 1 000µm, et en particulier de l'ordre de 750 µm.

Contrairement aux systèmes microfluidiques couramment développés par exemple pour le criblage enzymatique, on ne cherche pas à récupérer séparément chacune des spores isolées pour les caractériser, ni à les trier, puisque la population utilisée est identique génétiquement.

Ainsi, l'émulsion en sortie du dispositif microfluidique est récupérée dans son intégralité dans un récipient unique et est maintenue sous agitation pour éviter qu'elle ne casse. L'agitation peut par exemple être maintenue entre 50 et 300 tr/min, de préférence entre 120 et 150 tr/min, et plus préférentiellement de l'ordre de 120 tr/min entre 20 et 30°C.

### Germination

Une fois l'encapsulation réalisée, le procédé selon l'invention met en oeuvre une étape b) de germination des spores.

L'émulsion récupérée à l'issue de l'étape a) est maintenue sous agitation le temps nécessaire à la germination des spores, de préférence entre 1 et 40h, préférentiellement entre 10 et 30 heures, et plus préférentiellement encore environ 19h.

La germination est réalisée à température ambiante, en particulier entre 20 et 30°C, de préférence à 24°C.

Comme précédemment, l'agitation peut par exemple être maintenue entre 50 et 300 tr/min, de préférence entre 120 et 150 tr/min, et plus préférentiellement de l'ordre de 120 tr/min entre 20 et 30°C.

### Récupération des spores germées

Une fois la germination terminée, l'émulsion est déstabilisée de manière à récupérer une phase aqueuse contenant les spores germées (appelées tubes germinatifs), correspondant au début de formation du mycélium.

La fin de la germination est connue de l'homme du métier. Des données sont disponibles dans la littérature pour chaque champignon et une observation microscopique permet de le confirmer.

Tous les moyens connus de l'homme du métier pour casser l'émulsion peuvent être mis en oeuvre.

Selon un mode préféré de réalisation, la récupération des spores germées non agrégées est réalisée par déstabilisation de l'émulsion.

Par exemple, l'émulsion peut être rincée au moyen d'une phase huileuse dépourvue de tensioactif, jusqu'à ce que la concentration en tensioactif soit trop faible pour assurer la stabilité de la dispersion des gouttes de phase aqueuse dans l'huile. Les deux phases peuvent ensuite être aisément séparées.

La phase aqueuse ainsi récupérée peut être utilisée pour ensemencer une culture à plus grande échelle, comme une fiole, un fermenteur de propagation, voire un fermenteur de production.

### Mise en culture des spores germées

Le procédé selon l'invention comprend une dernière étape d) de mise en culture des spores germées en milieu liquide.

La mise en culture de l'étape d) est de préférence opérée sous agitation entre 20 et 30°C, de préférence 24°C, dans un milieu permettant la croissance de microorganismes filamenteux, de préférence milieu riche.

La mise en culture de l'étape d) dure de préférence entre 1h et 48h, et notamment environ 24h.

Le procédé selon l'invention permet d'obtenir, après généralement 24h de croissance, une culture fluide, non agrégée, de mycélium de champignons filamenteux ce qui présente un avantage pour leur mise en oeuvre industrielle (propagation facilitée, meilleure croissance par diffusion facilitée des nutriments au sein du mycélium).

La mise en culture peut être utilisée comme étape préliminaire à l'ensemencement d'un bioréacteur ou toute autre installation permettant la propagation et la croissance du champignon filamenteux encapsulé selon l'invention.

### EXEMPLE

Les exemples suivants illustrent l'invention.

### Exemple 1 : Protocole

### Phase aqueuse :

On a préparé une phase aqueuse composée de 15 mL d'une solution de milieu riche YPDP (Potatoes dextrose 24g/L, Yeast Extract 5g/L et Peptone 5g/L) diluée au demi et ensemencée par des spores de champignons à une concentration de 5.10⁵spores/mL, dans une seringue en verre.

Une agitation magnétique est mise en place dans la seringue pour éviter la sédimentation des spores. La banque de spores a été précédemment filtrée afin de s'affranchir de la présence éventuelle de mycélium.

### Phase huileuse :

On a préparé une phase huileuse composée de 30mL de solution d'huile fluorée FC3283 additionnée du surfactant biocompatible Picosurf à 3g/L, dans une seringue en verre.

### Dispositif expérimental :

Le dispositif expérimental est composé d'une puce microfluidique illustrée à la Figure 2, comprenant des canaux microfluidiques, obtenue par réticulation et collage de PDMS (RTV 615, société Momentive) sur une lame de verre par Plasma air (création de site actifs sous l'action d'un champ électrique Radio Fréquence qui entraine une forte adhérence entre les deux surfaces. Cette méthode consiste à activer la surface au plasma oxygène (ou air seul). Pour ce faire, un vide primaire est créé et un gaz excité est généré sous l'effet d'un champ électrique Radio-Fréquence. Les électrons et les ions à forte énergie qui constituent le plasma permettent de casser les liaisons de surface et de créer des sites "actifs", deux surfaces PDMS-PDMS ou PDMS-verre ainsi traitées peuvent alors créer des liaisons et adhérer fortement).

La puce microfluidique présentée à la Figure 2 est percée au niveau de l'arrivée des phases aqueuse et huileuse, et en sortie. Les conduits microfluidiques d'entrée ont un diamètre interne de 125 µm et celui de sortie de 750 µm.

Un traitement à l'OTS (octadécyltrichlorosilane) rend le système hydrophobe.

La puce microfluidique est placée sous un microscope inversé équipé d'une caméra permettant de visualiser en temps réel l'encapsulation. Les deux seringues contenant les phases aqueuse et huileuse sont placées sur deux pousse-seringues, permettant le réglage séparé des débits de chaque phase. L'émulsion en sortie de la puce microfluidique est recueillie dans un tube Falcon 50mL maintenu à une agitation constante de 120 tr/min durant toute la durée de la germination.

### Encapsulation :

La phase aqueuse contenant les spores est injectée à un débit de 3 mL/h, la phase huileuse contenant le surfactant à un débit de 8 mL/h. L'encapsulation dure 3h et permet d'obtenir environ 10 mL d'émulsion et 20 mL d'huile fluorée.

### Germination :

L'émulsion est maintenue, avec une agitation de 120 tr/min à 24°C, pendant 19 heures, temps nécessaire à la germination des spores.

### Déstabilisation de l'émulsion :

A la fin de l'étape de germination, l'émulsion est déstabilisée afin de récupérer uniquement la phase aqueuse contenant les spores germées. Pour se faire, des rinçages successifs à l'huile FC3283 dépourvue de surfactant sont réalisés :
L'huile résiduelle au fond du tube est prélevée, puis l'huile vierge est ajoutée en quantité équivalente à celle de l'émulsion (environ 10 mL). Le tube est agité manuellement pendant 5 min. Cette opération est répétée autant de fois que nécessaire jusqu'à observer une phase aqueuse claire et sans huile, contenant les spores germées.

### Croissance :

La phase aqueuse ainsi récupérée (8 mL environ) est ensuite transférée en Erlenmeyer de contenance 250 mL dans le milieu riche YPDP qsp 50 mL.

La croissance à 24°C sous agitation 175 tr/min dure 24h.

### Exemple 2 : application à Aspergillus oryzae

Les spores *d'Aspergillus oryzae,* espèce qui présente une morphologie de type agrégée en pellets formées par un mécanisme coagulant lors de sa culture en milieu riche liquide, sont encapsulées selon le protocole décrit dans l'exemple 1.

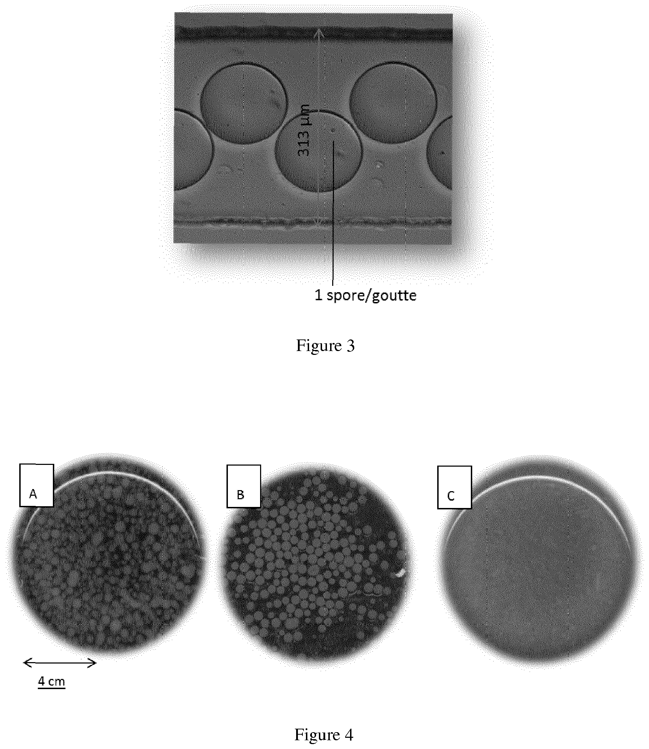

L'observation de l'étape d'encapsulation par le microscope (Figure 3) montre la présence d'en moyenne 1 spore/goutte et une taille de goutte d'environ 140 à 150 µm de diamètre (2 nL).

Deux témoins dont les spores ne sont pas encapsulées selon le procédé de l'invention sont également préparés :
- un témoin A comprenant 10 mL de milieu YPDP dilué au demi, ensemencé à 5.10⁵ spores/mL et placé en tube Falcon dans les même conditions que le tube de récupération de l'émulsion ; et
- un témoin B, identique au témoin A, additionné de 20 mL d'huile + tensioactif

Après mise en oeuvre du procédé décrit à l'exemple 1, les 10 mL d'émulsion déstabilisée selon l'invention contenant les spores germées sont transférés dans un Erlenmeyer contenant 40 mL de milieu riche YPDP. De la même manière, les deux témoins sont également transférés dans deux Erlenmeyer contenant 40 mL de milieu riche YPDP.

Au bout de 24h de croissance, la souche d'*Aspergillus oryzae* testée (NRRL 3488) montre les morphologies présentées à la Figure 4.

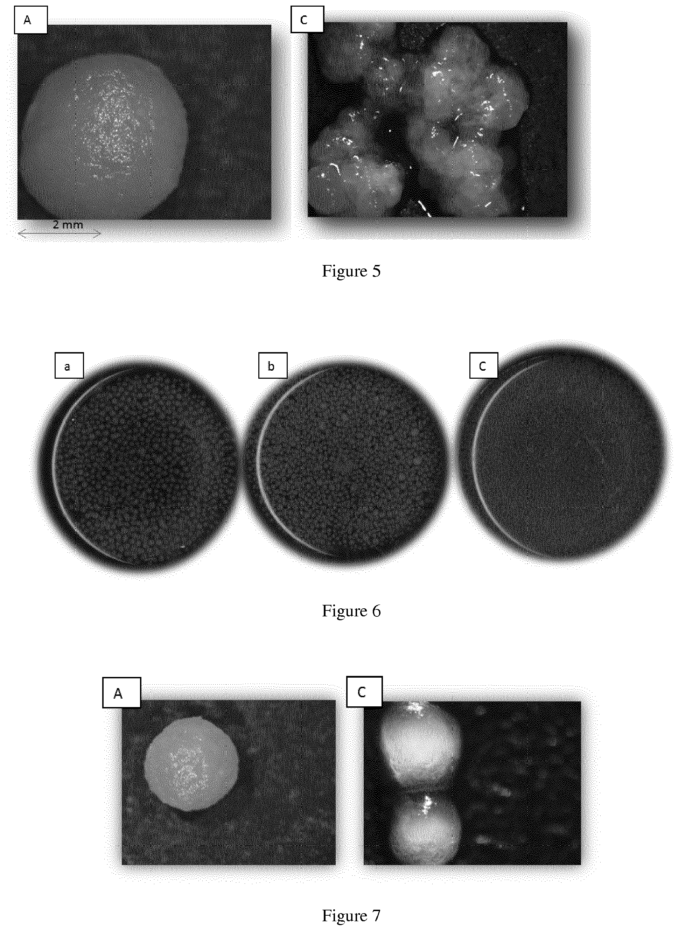

Les photos de la Figure 4 sont prises en scannant le font des Erlenmeyers contenant les champignons après 24h de culture. On observe que les photos A et B présentent un mycélium agrégé sous forme de « pellets » distinctes contrairement à la photo C où la culture est fluide sans agrégats. Les photographies prise en loupe binoculaire en Figure 5 montrent un grossissement de la culture A : grossissement d'une « pellet » et de la culture C : grossissement d'un échantillon fluide extrait de la culture C.

L'isolement des spores durant leur germination au moyen du procédé selon l'invention entraine un important changement morphologique de cette souche lors de sa croissance en milieu liquide.

### Exemple 3 : Application à Aspergillus terreus

Les spores d'*Aspergillus terreus*, espèce qui présente une morphologie de type agrégée en pellets formées par un mécanisme non-coagulant lors de sa culture en milieu riche liquide, sont encapsulées selon le protocole décrit dans l'exemple 1.

De la même manière que dans l'exemple 2, l'observation de l'étape d'encapsulation par le microscope montre la présence d'en moyenne 1 spore/goutte et une taille de goutte d'environ 140 à 150 µm de diamètre (2 nL).

Deux témoins dont les spores ne sont pas encapsulées selon le procédé de l'invention sont également préparés :
- un témoin A comprenant 10 mL de milieu YPDP dilué au demi, ensemencé à 5.10⁵ spores/mL et placé en tube Falcon dans les même conditions que le tube de récupération de l'émulsion ; et
- un témoin B, identique au témoin A, additionné de 20 mL d'huile + tensioactif

Après mise en oeuvre du procédé décrit à l'exemple 1, les 10 mL d'émulsion déstabilisée selon l'invention contenant les spores germées sont transférés dans un Erlenmeyer contenant 40 mL de milieu riche YPDP. De la même manière, les deux témoins sont également transférés dans deux Erlenmeyer contenant 40 mL de milieu riche YPDP.

Au bout de 24h de croissance, la souche d'*Aspergillus terreus* testée (NRRL 1960) montre les morphologies illustrées aux Figure 6 et Figure 7.

On observe que la morphologie de la souche d'*Aspergillus terreus* étudiée est agrégée sous forme de pellets dans les 2 cultures témoins (sans encapsulation, Figure 6A et B), mais également après encapsulation sous forme de pellets de diamètre légèrement inférieur (Figure6C).

Les photographies prises en loupe binoculaire en Figure 7 montrent un grossissement de la culture A : grossissement d'une « pellet » et de la culture C : grossissement de deux « pellets ».

Comme il apparait dans les présents exemples, le procédé selon l'invention permet une encapsulation de champignons filamenteux agrégés de type coagulants (par exemple la souche *Aspergillus oryzae*) mais ne donne pas de bons résultats pour des champignons de type non-coagulants (par exemple la souche *Aspergillus terreus*)

En effet, l'espèce *Aspergillus terreus* appartenant au type non-coagulant est capable de s'agréger après germination par agrégation des filaments mycéliens. La séparation physique des spores avant et pendant la germination ne permet donc pas d'empêcher cette espèce de présenter une morphologie agrégée en milieu liquide. *Aspergillus oryzae* appartenant au type coagulant ne s'agrège qu'aux stades précoces de son développement (avant et durant la germination), en séparant les spores par cette technique il n'y a plus d'agrégation possible pour cette espèce.

## Revendications

1. Procédé de modification de la morphologie de champignons filamenteux agrégés de type coagulant comprenant:
a) l'encapsulation des spores desdits champignons filamenteux dans la phase dispersée d'une émulsion eau-dans-huile,
b) la germination des spores encapsulées dans ladite phase dispersée de l'émulsion,
c) la récupération des spores germées non agrégées,
ladite émulsion étant obtenue par mise en oeuvre d'un dispositif microfluidique,
d) la mise en culture des spores germées en milieu liquide.

2. Procédé selon la revendication 1, **caractérisé en ce que** le champignon filamenteux de morphologie agrégée de type coagulant est choisi de préférence parmi les espèces *Aspergillus oryzae, Aspergillus niger ou Aspergillus nidulans*, et plus préférentiellement parmi les souches appartenant à l'espèce *Aspergillus oryzae.*

3. Procédé selon l'une quelconque des revendications 1 ou 2, **caractérisé en ce que** la phase dispersée de l'émulsion est une phase aqueuse comprenant au moins de l'eau, des spores de champignon à encapsuler en suspension dans un milieu de culture liquide.

4. Procédé selon l'une quelconque des revendications précédentes, **caractérisé en ce que** la phase huileuse de l'émulsion comprend au moins une huile biocompatible n'interférant pas dans la croissance de micro-organisme, de préférence au moins une huile fluorée.

5. Procédé selon la revendication 4, **caractérisé en ce que** la phase huileuse comprend au moins un tensioactif, de préférence biocompatible.

6. Procédé selon l'une quelconque des revendications précédentes, **caractérisé en ce que** le dispositif microfluidique comprend un premier conduit microfluidique alimenté en phase aqueuse contenant en suspension les spores à encapsuler, et un deuxième conduit microfluidique alimenté en phase huileuse, le premier conduit débouchant dans le deuxième conduit et formant une jonction fluidique avec ce dernier.

7. Procédé selon la revendication 6, **caractérisé en ce que** les premier et deuxième conduits microfluidiques ont une section pouvant s'inscrire dans un cercle de diamètre compris entre 1 µm et 1 mm ; de préférence entre 50 et 500 µm, et en particulier de l'ordre de 125 µm.

8. Procédé selon l'une quelconque des revendications 3 à 7, **caractérisé en ce que** la phase aqueuse de l' émulsion se présente sous la forme de gouttes dispersées dans la phase huileuse, lesdites gouttes présentant un diamètre compris entre 10 µm et 1 mm, de préférence entre 50 et 500 µm, et plus préférentiellement entre environ 140 et 150 µm.

9. Procédé selon l'une quelconque des revendications précédentes, **caractérisé en ce que** dispositif microfluidique d'encapsulation comprend, en outre :
- un moyen d'injection, dans le premier conduit dudit dispositif, d'une suspension formée par la phase aqueuse et par les spores à encapsuler, ledit moyen d'injection étant par exemple d'une seringue avec un pousse-seringue; et
- un moyen d'injection, dans le deuxième conduit du dispositif, de la phase huileuse; ledit moyen d'injection étant par exemple une seringue avec un pousse-seringue.

10. Procédé selon l'une quelconque des revendications 8 ou 9, **caractérisé en ce que** la concentration de spores dans la phase aqueuse et les débits d'injection des phases sont adaptés de manière à avoir en moyenne entre 1 et 10 spores par goutte, de préférence 1 spore par goutte.

11. Procédé selon l'une quelconque des revendications précédentes, **caractérisé en ce que** le dispositif microfluidique d'encapsulation comprend, en outre un conduit microfluidique de sortie, permettant la récupération de l'émulsion, présentant une section pouvant s'inscrire dans un cercle de diamètre compris entre 125 et 1 500µm, de préférence entre 500 et 1 000µm, et en particulier de l'ordre de 750 µm.

12. Procédé selon l'une quelconque des revendications précédentes, **caractérisé en ce que** l'étape b) de germination est opérée sous agitation à température ambiante, en particulier entre 20 et 30°C, de préférence à 24°C, le temps nécessaire à la germination des spores, de préférence entre 1 et 40h, préférentiellement entre 10 et 30 heures, et plus préférentiellement encore environ 19h.

13. Procédé selon l'une quelconque des revendications précédentes, **caractérisé en ce que** l'étape c) de récupération des spores germées est mise en oeuvre par déstabilisation de l'émulsion, de préférence par rinçage au moyen d'une phase huileuse dépourvue de tensioactif, jusqu'à ce que la concentration en tensioactif soit trop faible pour assurer la stabilité de la dispersion des gouttes de phase aqueuse dans la phase huileuse.

14. Procédé selon l'une quelconque des revendications précédentes, **caractérisé en ce que** l'étape d) de mise en culture des spores germées est opérée sous agitation entre 20 et 30°C, de préférence 24°C, dans un milieu permettant la croissance de microorganismes filamenteux, de préférence milieu riche.

15. Procédé selon l'une quelconque des revendications précédentes, **caractérisé en ce que** la mise en culture est utilisée comme étape préliminaire à l'ensemencement d'un bioréacteur ou toute autre installation permettant la propagation et la croissance du champignon filamenteux encapsulé.
